(19) Europäisches Patentamt
European Patent Office
Office européen des brevets

(11) Publication number: **0 033 488**
**A1**

(12) # EUROPEAN PATENT APPLICATION

(21) Application number: 81100464.7

(22) Date of filing: 22.01.81

(51) Int. Cl.[3]: C 07 D 213/803
C 07 D 491/056, A 61 K 31/455

(30) Priority: 22.01.80 IT 1935480

(43) Date of publication of application: 12.08.81 Bulletin 81/32

(84) Designated Contracting States: AT BE CH DE FR GB LI NL SE

(71) Applicant: D and D S.r.l.
Corso Venezia, 61
I-20121 Milano(IT)

(72) Inventor: Reiner, Alberto
Via Mentana 23
Como(IT)

(74) Representative: Weinhold, Peter, Dr. et al,
Siegfriedstrasse 8
D-8000 München 40(DE)

(54) **Therapeutically active derivatives of nicotinic acid, process for their preparation and related pharmaceutical compositions.**

(57) The novel derivatives of the nicotinic acid, having general formula:

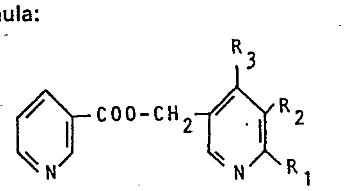

(1)

.HCl

wherein $R_1$ is H or methyl and $R_2$ and $R_3$, when separated, respectively represent OH and $CH_2OH$, or are combined in a cyclic structure forming the group:

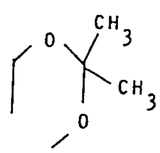

show analgesic and anti-inflammatory activity associated with specific effects, or hypoglycemic and hypochlolesteremic activity. The subject derivatives are prepared by reacting (3-4) isopropyliden pyridoxine with the chloride of the nicotinic acid.

EP 0 033 488 A1

The present invention relates to derivatives of the nicotinic acid, having the general formula:

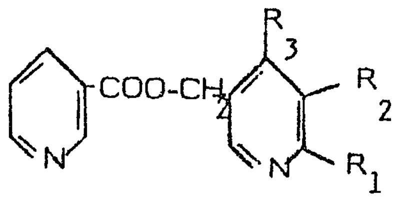

. HCl (1)

wherein $R_1$ is H or methyl, and $R_2$ and $R_3$, when separated, represent OH and $CH_2OH$ respectively, or are combined as a cyclic structure forming the group:

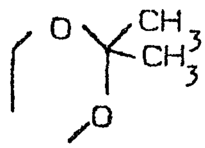

The subject compounds are thus monoesters of nicotinic acid with pyridoxine and (3-4) isopropyliden pyridoxine.
It has been particularly found that the hydrochloride of the (3-4) isopropyliden pyridoxine ester of nicotinic acid, having the formula:

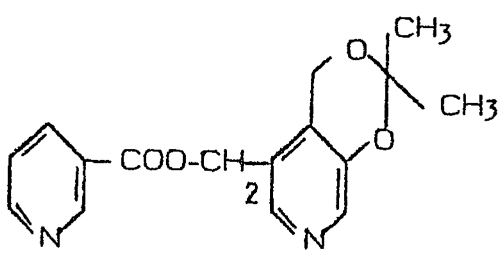

. HCl (2)

and the hydrochloride of the pyridoxine-5-ester of nicotinic acid, having the formula:

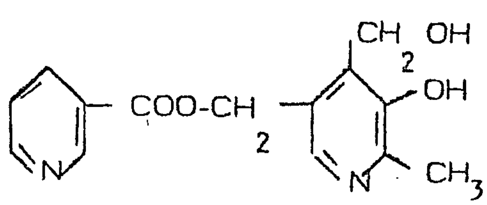

. HCl (3)

are endowed with interesting pharmacological properties by which they are useful for particular therapeutical uses. The compounds of the invention are in form of a white microcrystalline powder, partially soluble in water and substantially insoluble in organic solvents.

For the preparation of the compounds of the invention, the claimed process comprises a first step in which the (3-4) isopropyliden pyridoxine ester of nicotinic acid is prepared and a second step in which the desired pyridoxine-5-ester of nicotinic acid is obtained.

More specifically, according to the process of the invention hydrochloride of chloride of the nicotinic acid is reacted with triethylamine and (3-4) isopropyliden pyridoxine at the temperature of 50 - 55 °C under stirring, giving place to the reaction product as a base which is converted to the hydrochloride(nicotinic ester), the latter being subjected to acid hydrolysis to obtain the pyridoxine-5-ester of nicotinic acid, which is too obtained as the free base and then converted to the corresponding hydrochloride.

The following example describes, in an illustrative but non limiting sense, the preparation of the compounds of the invention.

Beispiel

a) (3-4) Isopropyliden pyridoxine ester of nicotinic acid

In a four neck flask, having a stirrer, cooling means, thermometer and dropping funnel, 24 g of hydrochloride of chloride of nicotinic acid and 200 mls of carbonium tetrachloride are charged, the flask being simultaneously cooled by an ice-water bath to maintain the temperature at a value not higher than 10 °C, preferably 5 °C.

Then 16,5 g of triethylamine are added, the cooling being continued, and thereafter the simultaneous addition of

further 16,5 g of triethylamine, and of 31,5 g of (3-4) isopropyliden pyridoxine takes place. At the end of the addition the temperature of the flask is brought to 50 - 55 °C and maintained thereto for about 2 hours under stirring, the reaction mass being then slowly cooled during about 12 hours. After a filtration for the separation of the triethylamine hydrochloride, the reaction mixture is concentrated under vacuum and the residual oil is taken with about 250 mls of methylisobutylketone. The desired product (32 g), namely (3-4) isopropyliden pyridoxine ester of nicotinic acid, crystallizes and is converted to the hydrochloride by treatment with gaseous hydrogen chloride in the stechiometrical ratio.

The product, having molecular weight 350.812 and melting point 153 - 157 °C (with dec.) is fairly soluble in alcohol, poorly soluble in water (it is hydrolyzed at pH 4) and insoluble in toluene and $CHCl_3$.
The analysis for $C_{17}H_{19}N_2O_4Cl$ gives:
calc. (%) : C 58.20; H 5.45; N 7.98; Cl 10.10
found (%) : C 58.00; H 5.10; N 7.85; Cl 9.95

b) Hydrochloride of the pyridoxine-5-ester of nicotinic acid

The compound prepared under (a) undergoes an acid hydrolysis with HCl in diluted aqueous solution, at the temperature of 60 °C for 2 hours. The base is cold precipitated with sodium carbonate for the product purification. The reaction product (25 g) is converted to the hydrochloride by treatment with HCl gas in acetone. The resulting product, having molecular weight 310.74 and melting point 151 - 153 °C, is poorly soluble in water and insoluble in ether and $CHCl_3$.
The analysis for $C_{14}H_{15}N_2O_4Cl$ gives:
calc. (%) : C 54.11; H 4,86; N 9.01; Cl 11.41
found (%) : C 54.50; H 4.30; N 9.40; Cl 11.10

Both compounds have been the subject of the preliminary pharmacological tests, by which it was first of all assessed that the toxicity of both compounds is low and does certainly fall within the limits allowed for a therapeutical use.

Turning now to the specific pharmacological properties, it has been found:

a) (3-4) Isopropyliden pyridoxine ester of nicotinic acid.

A remarkable diuretic activity is present, which (at dosages of 20 mg/kg of body weight) is comparable with that of the spironolactone.
A relevant analgesic activity is also present, which is comparable with that of the phenylbutazone at dosages of 200 mg/kg, but without symptoms of gastric irritation.
An anti-inflammatory activity (anti-oedema), comparable to that of aspirin at doses of 200 mg/kg is also observed.

Consequently this compound is endowed with anti-inflammatory and analgesic activity, without effects of gastric lesion.

b) Pyridoxine-5-ester of nicotinic acid

For this compound a specific hypoglycemic action has been assessed, which is comparable or even higher than that of phenformin. Furthermore, like other anti-inflammatory derivates, a slow and time delayed release of the nicotinic group can be foreseen.

As a consequence, for this compound a hypocholesterolemic activity is associated to the basic activity, namely the hypoglicemic activity. Both compounds can be used for pharmaceutical preparations, such as tablets, capsules, suppositories, creams, together with the conventional eccipients and vehicles, well known in the pharmaceutical art.

Claims

1.) Derivatives of nicotinic acid, having general formula:

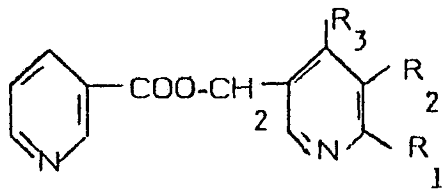

. HCl (I)

Wherein $R_1$ is H or methyl, and $R_2$ and $R_3$, when separated, respectively represent OH and $CH_2OH$, or are combined in a cyclic structure forming the group:

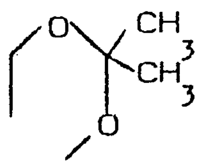

2.) Derivative according to claim 1, characterized in that it is the (3-4) isopropyliden-pyridoxine-5-ester of nicotinic acid or the mono hydrochloride thereof.

3.) Derivative according to claim 1, characterized in that it is the pyridoxine-5-ester of nicotinic acid or the hydrochloride thereof.

4.) A process for the preparation of the derivatives of claim 1, characterized in that hydrochloride of chloride of nicotinic acid is reacted with triethylamine and (3-4) isopropyliden pyridoxine at the temperature of 50 - 55 °C under stirring, giving the (3-4) isopropyliden pyridoxine ester of nicotinic acid and, if desired, is subjected to acid hydrolysis to pyridoxine-5-ester of nicotinic acid.

5.) A process according to claim 4, characterized in that the reactants are charged in the reaction tank at a temperature not higher than 10 °C, preferably not higher than 5 °C.

6.) A process according to claim 4, characterized in that the (3-4) isopropyliden-pyridoxine-5-ester of nicotinic acid is converted to the corresponding hydrochloride, by treatment with gaseous hydrogen chloride.

7.) A process according to claim 4, characterized in that the pyridoxine-5-ester of nicotinic acid is converted to the corresponding hydrochloride by reaction with gaseous hydrogen chloride.

8.) Pharmaceutical compositions with anti-inflammatory and analgesic activity characterized by containing, as the active ingredient, the derivative of claim 2, together with the conventional eccipients.

9.) Pharmaceutical composition with hypoglycemic and hypochloesterolemic activity, characterized by containing, as the active ingredient, the derivative of claim 3, together with the conventional eccipients.

0033488

European Patent Office

# EUROPEAN SEARCH REPORT

Application number

EP 81100464.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| X | US - A - 3 557 131 (YOSHIMURA)<br>+ Claims 1,4,7; columns 4, 5,8 +<br>-- | 1-4,9 | C 07 D 213/803<br>C 07 D 491/056<br>A 61 K 31/455 |
| X | GB - A - 1 070 120 (TANABE)<br>-- | 1-4,9 | |
| | US - A - 3 644 385 (UTSUMI)<br>+ Column 4; example 5 +<br>---- | 1-4 | |

TECHNICAL FIELDS SEARCHED (Int. Cl.³)

C 07 D 213/00
C 07 D 491/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 06-04-1981 | PETROUSEK |

EPO Form 1503.1 06.78